(19) Europäisches Patentamt
European Patent Office
Office européen des brevets

(11) EP 0 531 487 B1

(12) **EUROPÄISCHE PATENTSCHRIFT**

(45) Veröffentlichungstag und Bekanntmachung des Hinweises auf die Patenterteilung:
**10.01.1996 Patentblatt 1996/02**

(21) Anmeldenummer: **92906987.0**

(22) Anmeldetag: **26.03.1992**

(51) Int. Cl.⁶: **A61L 25/00**, A61L 27/00

(86) Internationale Anmeldenummer: **PCT/DE92/00245**

(87) Internationale Veröffentlichungsnummer: **WO 92/17217 (15.10.1992 Gazette 1992/26)**

(54) **VERWENDUNG EINER MISCHUNG FÜR DIE HERSTELLUNG VON MEDIZINISCHEN IMPLANTATEN**

USE OF A MIXTURE TO PRODUCE MEDICAL IMPLANTS

UTILISATION D'UN MELANGE POUR LA FABRICATION D'IMPLANTS MEDICAUX

(84) Benannte Vertragsstaaten:
**CH DE FR GB LI**

(30) Priorität: **28.03.1991 DE 4110316**

(43) Veröffentlichungstag der Anmeldung:
**17.03.1993 Patentblatt 1993/11**

(73) Patentinhaber:
- **STORCH, Uwe**
**D-45889 Gelsenkirchen (DE)**
- **STANKEWITZ, Bernd**
**D-52062 Aachen (DE)**

(72) Erfinder:
- **STORCH, Uwe**
**D-45889 Gelsenkirchen (DE)**
- **STANKEWITZ, Bernd**
**D-52062 Aachen (DE)**

(74) Vertreter: **Haft, von Puttkamer, Berngruber, Czybulka**
**D-81669 München (DE)**

(56) Entgegenhaltungen:
**EP-A- 0 280 451**
**EP-A- 0 332 405**
**EP-A- 0 466 552**
**DE-A- 3 500 287**
**US-A- 4 477 604**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 531 487 B1

Printed by Rank Xerox (UK) Business Services
2.9/3.4

## Beschreibung

Die Erfindung betrifft die Herstellung von medizinischen Implantaten im menschlichen und tierischen Körper.

Es ist bekannt, medizinische Implantate aus Festkörpern auf Basis von Polyurethan herzustellen, wobei das Implantat zunächst seiner Form nach dem späteren Einsatzort entsprechend aus dem Festkörper herausgearbeitet und anschließend in den menschlichen oder tierischen Körper implantiert wird. Das ist in erheblichem Umfang aufwendig und oftmals auch nicht realisierbar, weil die Form des Implantationsortes oftmals nicht feststellbar ist.

Der Erfindung liegt die Aufgabe zugrunde, die Anpassung des Implantates an den Implantationsort zu vereinfachen.

Zur Lösung dieser Aufgabe lehrt die vorliegende Erfindung die Verwendung einer zu einem körperverträglichen, biologisch resorbierbaren Festkörper reagierenden pastösen Mischung aus Polyurethan bildenden Komponenten und Hydroxylapitat als Füllstoffen für die Herstellung von medizinischen Implantaten im menschlichen und tierischen Körper, insbesondere zur Auffüllung parodontaler Knochentaschen, Augmentation am Kieferknochen, endodontischen Füllung und Beseitigung von Knochendefekten.

Der durch die Erfindung erreichte Vorteil besteht darin, daß durch den Einsatz der pastösen bzw. plastischen Mischung sich das Implantat dem Implantationsort gleichsam von selbst anpassen kann und danach zum Festkörper ausreagiert. Dieser Festkörper kann anschließend biologisch resorbiert werden. Zur Herstellung und Verarbeitung der Mischung bieten sich zwei Verfahren an. Einerseits können alle Komponenten in einem Arbeitsgang zusammengeführt und dann verarbeitet werden, auf der anderen Seite kann aber auch mit einem Präpolymer gearbeitet werden. Jedenfalls können in allen Fällen über die Zusammensetzung der Mischung die verarbeitungstechnischen, werkstoffkundlichen und anwendungsspezifischen Eigenschaften, wie z.B. die Topfzeit, das Molekulargewicht und der Abbau, beeinflußt werden. Das gilt auch für die Füllstoffe, die rein physikalisch und/oder im Sinne von Blends durch intermolekulare Wechselwirkungen in die Polyurethanmatrix eingebunden werden können oder im Sinne von Grafts an die Hauptkette angelagert werden können.

Für die weitere Ausgestaltung bestehen im Rahmen der Erfindung mehrere Möglichkeiten. So liegt nach einer bevorzugten Ausführungsform das Massenverhältnis von das Polyurethan bildenden Komponenten zu Füllstoffen zwischen 1 : 3 und 1 : 1. Die das Polyurethan bildenden Komponenten sollten aus

Toluendiisozyanat,
Methylen-bis-(p-Phenyl)diisozyanat,
Hexandiisozyanat,
Methylen-bis-(p-cyclohexyl)diisozyanat
und/oder
Isophorondiisozyanat
2.2.4/2.4.4-Trimethylhexamethylendiisozyanat

als Diisozyanatkomponente und

Polyester, Polyether, Polycaprolacton, Polysiloxan, Castoröl, Butandiol, Hexandiol, Ethylenglykol, Polytetramethylenoxid und/oder Ethylendiamin

als Diolkomponente bestehen. Als Diisozyanatkomponente sind dabei die aliphatischen vorzuziehen; bei der Diolkomponente sind das Castoröl, Polytetramethylenoxid und Ethylendiamin. Wie schon gesagt, können die das Polyurethan bildenden Komponenten ohne weiteres als Präpolymer eingesetzt werden.

Als Füllstoffe haben sich in der Praxis besonders Hydroxylapatit und Polylactid-Oligomer bewährt. Das Hydroxylapatit ist zweckmäßigerweise in Pulver- oder Granulatform der Mischung beigegeben. Dabei kann dieses Hydroxolapatit silanisiert sein, wodurch eine Einbindung in die Polyurethanmatrix möglich ist, oder porös sein, was die biologische Aktivität steigert; mit einer Porengröße im Bereich von 200 µm ist ein solches Hydroxylapatitgranulat als Knochenersatzmaterial handelsüblich (Interpore 200). Das der Mischung beigegebene Polylactid-Oligomer ist zweckmäßigerweise faserförmig; es läßt sich durch Schmelzspinnen mit einem Durchmesser von 50 bis 250 µm herstellen.

Nach weiteren bevorzugten Ausführungsformen wird mit Tetracyclin als antibiotisch wirkendem Füllstoff und/oder mit wenigstens einer barischen Aminosäure, insbes. Lysin, als zusätzlichem Vernetzungsmittel gearbeitet.

Bezüglich der Mechanismen zur Steuerung der Abbaurate ist zu bemerken, daß die Anfälligkeit eines Polyurethans gegenüber den einzelnen Abbaumechanismen stark von seiner Zusammensetzung abhängig ist und daher über diese gesteuert werden kann. So wird die Hydrolysebeständigkeit durch die verwendete Polyol-Komponente bestimmt. Versuche hierzu haben folgende Rangfolge in der Hydrolysestabilität ergeben:

Polyether > Polycaprolactone > Polyester

Der hydrolytische Zerfall der Estergruppe wird durch saures Milieu begünstigt und verläuft wie folgt:

$$\sim\!-\overset{\overset{\displaystyle O}{\|}}{C}-O-\!\sim \; + \; H_2O \rightarrow \sim\!-OH \; + \; HO-\overset{\overset{\displaystyle O}{\|}}{C}-\!\sim$$

Dadurch, daß beim Hydrolysevorgang endständige Carbonsäuregruppen entstehen, ist der Vorgang autokatalytisch. Bei Polyetherurethanen wird vorwiegend die Urethangruppe selbst hydrolysiert. Dabei entstehen zwei kürzere Ketten. Die eine besitzt eine endständige Hydroxylgruppe, die andere endet aminofunktionell.

$$\sim(O\text{-}CH_2)_4)_n)\text{-}O\text{-}CO\text{-}NH\text{-}R\text{-}NH\text{-}CO\text{-}O\sim + H_2O \rightarrow \sim(O\text{-}(CH_2)_4)_n\text{-}OH + H_2N\text{-}R\text{-}NH\text{-}CO\text{-}O\sim + CO_2$$

Durch den Zerfall von beigefügtem Polylactid-Oligomer werden in Abhängigkeit von der Kettenlänge desselben und seines Massenanteiles diese Prozesse beschleunigt. Gleiches gilt allgemein für die Freisetzung von Säuren, z.B. Phosphorsäure aus Hydroxylapatit. Durch oben beschriebene Mechanismen sowie Vernetzungsgrad, Kristallinität, Porengröße und Polymerisationsgrad ist die Degradationsgeschwindigkeit des Werkstoffes einstellbar.

Im folgenden wird die Erfindung anhand einiger Beispiele näher erläutert:

Beispiel 1: Auffüllung parodontaler Knochentaschen

Die parodontale Knochentasche entsteht im Rahmen fortgeschrittener parodontaler Erkrankungen als Folge von bakteriellen, wiederkehrenden. Infektionen. Sie liegt im Grenzbereich zwischen Zahnwurzelzement, Faserapparat (desmodontale Fasern), Knochen und Bindegewebe (angeheftete Gingiva). Die operative Behandlung beginnt mit der Entfernung der pathogenen Keime, deren Konkrementen und des Granulationsgewebes. Die Regenerationsfähigkeit aller einzelnen oben genannten Gewebe ist durch unterschiedliche Verfahren medizinisch nachgewiesen. Nach operativer Darstellung finden sich die unterschiedlichsten Defekte, die in ihrer Vielgestaltigkeit nur durch ein plastisch einbringbares Material zufriedenstellend aufzufüllen sind. Die Gewebsregeneration wird durch die unterschiedlichen Degenerationszeiten der einzelnen Komponenten des Implantatmaterials gesteuert. Eine sinnvolle Zusammensetzung für diesen Einsatzzweck ist:

| | |
|---|---|
| 40 Massenteile | Isophorondiisozyanat und Castoröl im Molverhältnis 4 : 1 als Präpolymer |
| 10 Massenteile | Hydroxylapatit-Pulver (Korngröße < 80 µm) |
| 20 Massenteile | silanisiertes Hydroxylapatit-Pulver |
| 15 Massenteile | Hydroxylapatit-Granulat (Interpore 200) |
| 15 Massenteile | Polylactid-Oligomer-Fasern (d = 50 - 250 µm; MW ca. 11 000) |

Die Polyurethan-Matrix des Compounds dient dem Zweck, die Zusätze zu binden, und nach der Aushärtung vor Ort als Festkörper zu stabilisieren. Desweiteren entwickelt sie eine starke Adhäsion an die Umgebung, welche auf die Verwendung von Castoröl zurückzuführen ist. Die Polylactid-Oligomer-Fasern dienen durch ihren frühzeitigen Zerfall innerhalb des Gemisches der gerichteten Gewebsregeneration. Das nach dem Polylactid-Oligomer-Zerfall entstandene Netz aus Hohlräumen ähnelt dem trabekulären Aufbau des Knochens. Da diese Hohlräume zuerst durch neugebildeten Knochen ersetzt werden, findet ein rasches Durchwachsen des Implantates statt. Hydroxylapatit ist ein klinisch bewährtes Knochenanalogon. In der handelsüblichen Form Interpore 200 weist es eine bioaktive Oberflächenbeschaffenheit auf. Das silanisierte Hydroxylapatit-Pulver ist über ein aminofunktionelles Silan (3-Triethoxysilylpropylamin) chemisch in die Matrix eingebunden. Durch diesen Effekt wird das Netzwerk für die Anwendungsdauer stabilisiert.

Die adhäsiven Eigenschaften des Materials ermöglichen eine gute Stabilisierung des marginalen Fibrinkoagels sowie eine Anheftung des Mukoperiostlappens. Dieses garantiert komplikationslosere Wundheilungsvorgänge. Bioaktive Oberflächen sorgen für die Ausbildung funktionell ausgerichteter desmodontaler Faserbündel. Als Heilungsergebnisse finden sich desweiteren eine Osteoneogenese, Zementbildung und eine Anheftung des marginalen Parodonts.

Beispiel 2: Augmentation am Kieferknochen

Der Verlust des Alveolarfortsatzes bei längerer Zahnlosigkeit macht eine suffiziente Prothetik (Zahnersatz) unmöglich und erfordert eine Augmentation am oder eine Implantation in den Knochen. Neben den bisher beschriebenen Eigenschaften ist eine Langzeitstabilität der Matrix erforderlich, da der neugebildete Knochen alleine wieder starken Resorptionsprozessen unterliegen würde. Angestrebt ist daher eine Verzapfung des Aufbaus an die Basis. Eine sinnvolle Zusammensetzung für diesen Zweck ist:

| | |
|---|---|
| 45 Massenteile | Polytretramethylenoxid, Castoröl und Isophorondiisozyanat im Molverhältnis 1 : 1 : 8 als Präpolymer |
| 15 Massenteile | Hydroxylapatit-Pulver |
| 20 Massenteile | silanisiertes Hydroxylapatit-Pulver |
| 10 Massenteile | Polylactid-Oligomer-Fasern |
| 10 Massenteile | Polylactid-Oligomer-Pulver (Korngröße ca. 600 µm) |

Der Polyurethan-Anteil dient auch hier dem Zweck, als Matrixsubstanz die Füllstoffpartikel zu verbinden und zu halten. Die adhäsiven Eigenschaften fixieren das aufgetragene Material. Durch den erhöhten Polylactid-Oligomer-Anteil wird das anfängliche Einwachsen des Knochengewebes begünstigt. Die durch Einsatz von Polytetramethylenoxid und gesteigerten Hydroxylapatit-Silan-Anteil reduzierte Abbaurate der Matrix stellen die angestrebte Funktion des Verbundes Knochen-Implantat sicher.

Beispiel 3: Endodontisches Füllungsmaterial

Das Anforderungsprofil umfaßt hier mehrere Punkte, nämlich (a) Ausfüllung sehr kleiner, dreidimensional verzweigter Kanäle und Kavitäten, (b) hohe Randdichtigkeit, (c) leichte Einführbarkeit, (d) antiseptiscne bakteriostatische Wirkung, (e) röntgenpositive Reaktion und (f) die Verweildauer des Zahnes überschreitende Resorptionszeit. Eine geeignete Mischungsvariante ist:

| | |
|---|---|
| 70 Massenteile | Methylen-bis-(p-cyclohexyl)diisozyanat, Polytetramethylenoxid und Ethylendiamin im Molverhältnis 4 : 1 : 3 |
| 25 Massenteile | Thoriumoxid (Kontrastmittel) |
| 5 Massenteile | Tetracyclin (Antibiotikum) |

Zur Erfüllung der Forderungen (a) bis (c) ist eine Volumenexpansion in der Aushärtungsphase eines anfänglich niedrigviskosen Materials erforderlich. Obige Mischung wird diesem Anspruch gerecht, falls der Wurzelkanal mit $H_2O_2$ vor Applikation des Materials gespült wurde. Die Freisetzung von Tetracyclin wirkt bakteriostatisch. Thoriumoxid genügt dem Anspruch unter (e).

Eine Polyurethan-Matrix in obiger Zusammensetzung weist einen hohen Kristallinitätsgrad auf, wodurch die geforderte Langzeitstabilität nach (f) erfüllt wird.

Beispiel 4: Herstellung des Implantatwerkstoffes

Der Implantatwerkstoff kann nach der Präpolymermethode, aber auch in einem Ein-Schritt-Verfahren hergestellt werden.

Bei der Präpolymermethode werden zunächst 1 g der Castorol-Komponente und 0,7 g Isophorondiisozyanat in ein Reagenzglas eingewogen. Dieses wird danach in ein auf 60 °C vorgewärmtes Ultraschallbad gestellt, in dem das Gemisch in 12 Stunden zum Präpolymer ausreagiert. Es läßt sich mittels Elektronenstrahl sterilisieren. Das Präpolymer ist nun gebrauchsfähig.

In eine sterile Petrischale werden 0,2 g Hydroxylapatit, 0,3 g silanisiertes Hydroxylapatit und 0,4 g Interpore 200 unter Praxisbedingungen eingewogen. Dazu kommt 0,8 g des Präpolymeren und alle Bestandteile werden unter Zugabe von 0,2 ml 4molearer NaOH sorgfältig vermischt. Nun werden 0,3 g der Polylactid-Oligomer- Fasern beigemengt. Danach ist das Material anwendungsbereit.

Beim Ein-Schritt-Verfahren werden in einem Reagenzglas 1,5 g Thoriumoxid und 3 ml Methylen-bis-(p-cyclohexyl) diisozyanat vermischt. In einem zweiten Reagenzglas wird 1 ml Polytetramethylenoxid mit 0,6 ml Ethylendiamin und 0,4

g Tetracyclin verrührt. Die Mischungen können dann in die getrennten Behälter einer Zweikomponenten-Spritze aufgezogen und appliziert werden.

## Patentansprüche

1. Verwendung einer zu einem körperverträglichen, biologisch resorbierbaren Festkörper reagierenden pastösen Mischung aus Polyurethan-bildenden Komponenten und Füllstoffen für die Herstellung von medizinischen Implantaten im menschlichen und tierischen Körper, insbesondere zur Auffüllung parodontaler Knochentaschen, Augmentation am Kieferknochen, endodontischen Füllung und Beseitigung von Knochendefekten, dadurch gekennzeichnet, daß als Füllstoff Hydroxylapatit eingesetzt wird.

2. Verwendung nach Anspruch 1, wobei das Massenverhältnis von das Polyurethan-bildenden Komponenten zu Füllstoffen zwischen 1 : 3 und 1 : 1 liegt.

3. Verwendung nach Anspruch 1, wobei die das Polyurethanbildenden Komponenten aus
   Toluendiisocyanat,
   Methylen-bis-(p-phenyl)diisocyanat,
   Hexandiisocyanat,
   Methylen-bis-(p-cyclohexyl)diisocyanat
   und/oder
   Isophorondiisocyanat,
   2.2.4/2.4.4-Trimethylhexamethylendiisocyanat
   als Diisocyanatkomponente und
   Polyester, Polyether, Polycaprolacton, Polysiloxan, Castoröl, Butandiol, Hexandiol, Ethylenglykol, Polytetramethylenoxid und/oder Ethylendiamin
   als Diolkomponenten bestehen.

4. Verwendung nach einem der Ansprüche 1 bis 3, wobei die das Polyurethan-bildenden Komponenten als Präpolymer eingesetzt werden.

5. Verwendung nach Anspruch 1, wobei der Hydroxylapatit silanisiert oder porös ist.

6. Verwendung nach einem der vorstehenden Ansprüche, wobei als Füllstoff zusätzlich ein Polylactid-Oligomer eingesetzt wird.

7. Verwendung nach Anspruch 6, dadurch gekennzeichnet, daß das Polylactid-Oligomer faserförmig ist.

8. Verwendung nach einem der vorstehenden Ansprüche, wobei der Mischung aus Polyurethan-bildenden Komponenten und Füllstoffen Tetracyclin zugesetzt ist.

9. Verwendung nach einem der vorstehenden Ansprüche, wobei wenigstens eine basische Aminosäure, insbesondere Lysin, als zusätzliches Polyurethan-Vernetzungsmittel eingesetzt wird.

## Claims

1. Use of a pasty mixture of polyurethane forming components and filling materials, which reacts to a physically compatible biologically resorbable solid material, for the production of medical implants in human and in animal bodies, in particular for filling parodontal bone pockets, augmentation of jaw bones, endodontic filling and the correction of bone defects, characterised in that hydroxylapatite is used as a filling material.

2. Use according to claim 1, wherein the mass ratio of the polyurethane forming components to filling materials is between 1:3 and 1:1.

3. Use according to claim 1, wherein the polyurethane forming components consist of
   toluene diisocyanate,
   methylene-bis-(p-phenyl)diisocyanate,
   hexandiisocyanate,
   methylene-bis-(p-cyclohexyl)diisocyanate
   and/or

isophorone diisocyanate,
2.2.4/2.4.4-trimethylhexamethylene diisocyanate
as diisocyanate components and
polyesters, polyethers, polycaprolactone, polysiloxane, castor oil, butandiol, hexandiol, ethylene glycol, polytetramethylene oxide and/or ethylenediamine
as diol components.

4. Use according to one of claims 1 to 3, wherein the polyurethane forming components are used as prepolymers.

5. Use according to claim 1, wherein the hydroxylapatite is silanized or porous.

6. Use according to one of the preceding claims, wherein a polylactide oligomer is additionally used as filling material.

7. Use according to claim 6, characterised in that the polylactide oligomer is fibrous.

8. Use according to one of the preceding claims, wherein tetracylin is added to the mixture of polyurethane forming components and filling materials.

9. Use according to one of the preceding claims, wherein at least a basic amino acid, in particular lysine is used as an additional polyurethane cross-linking agent.

**Revendications**

1. Utilisation d'un mélange pâteux réagissant sur un solide supportable par le corps et biologiquement résorbable, le mélange comprenant des éléments de formation de polyuréthanne et des charges pour la réalisation d'implants médicaux dans le corps humain et animal, notamment pour le remplissage de cavités osseuses parodontales, le regarnissage de l'os maxillaire, le remplissage et l'élimination endodontique de défauts osseux, caractérisée en ce que l'hydroxylapatite est employée en tant que charge.

2. Utilisation selon la revendication 1, dans laquelle la proportion massique des éléments de formation de polyuréthanne par rapport aux charges est comprise entre 1:3 et 1:1.

3. Utilisation selon la revendication 1, dans laquelle les éléments de formation de polyuréthanne comprennent un diisocyanate parmi :
le diisocyanate de toluène,
le méthylène-bis-(p-phényl)diisocyanate,
le diisocyanate d'hexane,
le méthylène-bis-(p-cyclohexyl)-diisocyanate et/ou
le diisocyanate d'isophorone,
le 2.2.4/2.4.4-triméthylhexaméthylène-diisocyanate
et comprennent un diol parmi
le polyester, le polyether, le polycaprolacton,
le polysiloxane, l'huile de castor, le butanediol,
l'hexanediol, l'éthylène-glycol, l'oxyde de polytétraméthylène, et/ou l'éthylenediamine.

4. Utilisation selon l'une des revendications 1 à 3, dans laquelle les éléments de formation de polyuréthanne sont employés en tant que prépolymère.

5. Utilisation selon la revendication 1, dans laquelle l'hydroxylapatite est silanisée ou poreuse.

6. Utilisation selon l'une quelconque des revendications précédentes, dans laquelle un oligomère de polylactide est également employé en tant que charge.

7. Utilisation selon la revendication 6, caractérisée en ce que le polylactide-oligomère est sous forme de fibres.

8. Utilisation selon l'une quelconque des revendications précédentes, dans laquelle la tétracycline est ajoutée au mélange d'éléments de formation de polyuréthanne et de charges.

**9.** Utilisation selon l'une quelconque des revendications précédentes, dans laquelle au moins un acide aminique de base, notamment la lysine, est employé en tant qu'agent de réticulation supplémentaire du polyuréthanne.